(19) Europäisches Patentamt
European Patent Office
Office européen des brevets

(11) EP 0 739 622 B1

(12) # EUROPÄISCHE PATENTSCHRIFT

(45) Veröffentlichungstag und Bekanntmachung des Hinweises auf die Patenterteilung:
**20.06.2001 Patentblatt 2001/25**

(51) Int Cl.7: **A61K 7/13**

(21) Anmeldenummer: **95119004.0**

(22) Anmeldetag: **02.12.1995**

(54) **Haarfärbemittel**

Hair dye composition

Composition de teinture pour cheveux

(84) Benannte Vertragsstaaten:
**DE ES FR GB IT**

(30) Priorität: **29.04.1995 DE 19515903**

(43) Veröffentlichungstag der Anmeldung:
**30.10.1996 Patentblatt 1996/44**

(73) Patentinhaber: **Wella Aktiengesellschaft
64274 Darmstadt (DE)**

(72) Erfinder:
- **Balzer, Wolfgang R., Dr.
D-64665 Alsbach (DE)**
- **Braun, Hans-Jürgen, Dr.
CH-3182 Ueberstorf (DE)**

(56) Entgegenhaltungen:
**EP-A- 0 395 837** **WO-A-93/25182**

Bemerkungen:
Die Akte enthält technische Angaben, die nach dem Eingang der Anmeldung eingereicht wurden und die nicht in dieser Patentschrift enthalten sind.

EP 0 739 622 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Gegenstand der Erfindung ist ein Mittel zum oxidativen Färben von Haaren auf der Basis einer Entwicklersubstanz/Kupplersubstanz-Kombination und eines gelben bis gelb-orangen Azofarbstoffes.

In der Haarfärbepraxis haben Oxidationsfarbstoffe eine wesentliche Bedeutung erlangt. Hierbei werden die Farbstoffe durch oxidative Kupplung von Entwicklersubstanzen mit Kupplersubstanzen im Haarschaft erzeugt. Dies führt zu sehr intensiven Haarfärbungen mit sehr guter Farbechtheit.

Als Entwicklersubstanzen werden bevorzugt 2,5-Diaminotoluol, 1,4-Diaminobenzol, 2-(2'-Hydroxyethyl)-1,4-diaminobenzol, 4-Aminophenol, 4-Amino-2-aminomethylphenol, 4-Amino-3-methylphenol sowie substituierte 4,5-Diaminopyrazole verwendet.

Die bevorzugt verwendeten Kupplersubstanzen sind dabei m-Phenylendiamin und dessen Derivate, wie zum Beispiel 2,4-Diamino-phenoxyethanol, 2,4-Diamino-5-fluor-toluol und 2-Amino-4-(2'-hydroxyethyl)-aminoanisol, oder Pyridinderivate, wie zum Beispiel 3,5-Diamino-2,6-dimethoxypyridin, als Blaukuppler, 1-Naphthol, m-Aminophenol und dessen Derivate, wie zum Beispiel 2-Amino-4-chlor-6-methyl-phenol, 5-Amino-2-methylphenol, 4-Amino-2-hydroxyphenoxyethanol, 4-Amino-5-fluor-2-hydroxy-toluol und 4-Amino-5-ethoxy-2-hydroxy-toluol, als Rotkuppler sowie Resorcin, 4-Chlorresorcin, 4,6-Dichlorresorcin, 2-Methylresorcin, 4-Hydroxy-1,2-methylendioxybenzol, 4-(2'-Hydroxyethyl)amino-1,2-methylendioxybenzol und 4-Hydroxyindol als Kuppler für den Braun-Blond-Bereich.

Durch eine entsprechende Kombination der einzelnen Entwickler und Kuppler kann eine Vielzahl unterschiedlicher Farbnuancen erzielt werden, die in der Regel die an Haaranfärbungen gestellten Erfordernisse erfüllen.

Bestimmte Nuancen können hingegen mit Oxidationshaarfärbemitteln nur sehr schwer erzielt werden. So ist es beispielsweise äußerst schwierig, modische Nuancen im Goldbereich mit Oxidationsfarbstoffen zu erzielen.

Je nach Haarstruktur und Färbebedingungen, welche in der Praxis zwangsläufig gewissen Schwankungen unterliegen, treten bei Haaranfärbungen mit Oxidationshaarfärbemitteln Farbverschiebungen auf. Die hieraus resultierenden unterschiedlichen Färbeergebnisse, welche in der Regel zu grünstichig beziehungsweise orangestichig sind, sind für die Kunden unbefriedigend und teilweise nicht akzeptabel.

Es bestand daher die Aufgabe, Haarfärbemittel zur Verfügung zu stellen, die eine Vielzahl von Farbnuancen einschließlich der modischen Nuancen im Goldbereich ermöglichen, wobei die vorgenannten Farbverschiebungen gar nicht oder nur in sehr geringem Umfang auftreten.

Es wurde nunmehr überraschenderweise gefunden, daß die vorgenannte Aufgabe durch ein Haarfärbemittel, welches eine Entwicklersubstanz/Kupplersubstanz-Kombination sowie bestimmte gelbe bis gelb-orange Azofarbstoffe enthält, in hervorragender Weise gelöst wird.

Gegenstand der vorliegenden Erfindung ist daher ein Mittel zur oxidativen Färbung von Haaren auf der Basis einer Entwicklersubstanz-/Kupplersubstanz-Kombination, dadurch gekennzeichnet, daß es mindestens einen Azofarbstoff der Formel (I),

$$A - N = N - B \qquad (I),$$

in der A einen p-Aminophenylrest der Formel (II) oder einen 3-Pyridylrest der Formel (III) darstellt

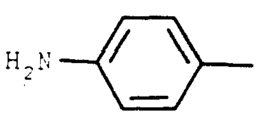
(II)

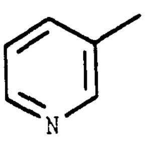
(III)

und B einen 4-[N,N-Bis-(2'-hydroxyethyl)amino]-phenylrest der Formel (IV) oder einen 2,6-Diamino-3-pyridylrest der Formel (V)

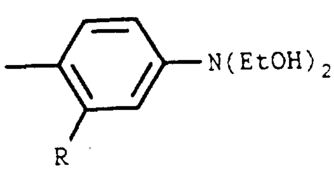
(IV),

wobei R gleich Wasserstoff, einer Alkylgruppe mit 1 bis 6 Kohlenstoffatomen, Fluor, Chlor, Brom oder Jod ist,

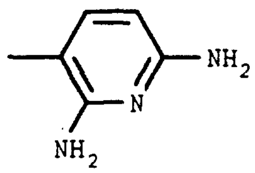

(V)

bedeutet, enthält.

Unter den Azofarbstoffen der Formel (I) sind solche bevorzugt, bei denen der Rest A eine Gruppe gemäß Formel (II) oder (III) darstellt wenn der Rest B gleich Formel (V) ist oder der Rest A eine Gruppe gemäß Formel (II) darstellt wenn der Rest B gleich Formel (IV) ist.

Unter diesen Azofarbstoffen sind die folgenden Verbindungen besonders bevorzugt:

4-Amino-4'-[N,N-bis-(2"-hydroxyethyl)amino]azobenzol, 4-Amino-2'-methyl-4'-[N,N-bis-(2"-Hydroxyethyl) amino]azobenzol, 4-Amino-2'-chlor-4'-[N,N-bis-(2"-Hydroxyethyl)amino]azobenzol und 2,6-Diamino-3-[3'-azopyridiyl]-pyridin.

In dem Haarfärbemittel können die Azofarbstoffe der Formel (I) sowohl einzeln als auch im Gemisch miteinander eingesetzt werden, wobei die Gesamteinsatzmenge vorzugsweise etwa 0,01 bis 2 Gewichtsprozent beträgt. Besonders bevorzugt ist hierbei eine Einsatzmenge des Azofarbstoffes der Formel (I) von etwa 0,05 bis 0,5 Gewichtsprozent.

Von den Entwicklersubstanzen kommen als Bestandteil des erfindungsgemäßen Haarfärbemittels insbesondere 1,4-Diaminobenzol, 2,5-Diaminotoluol, 2-(2'-Hydroxyethyl)-1,4-diaminobenzol, 4-Aminophenol, 4-Amino-2-aminomethylphenol, 4-Amino-3-methylphenol, 4-Amino-2-methoxymethyl-phenol, 4-Amino-2-ethoxymethyl-phenol, 4,5-Diamino-1-methyl-pyrazol, 4,5-Diamino-1-isopropylpyrazol, 1-Benzyl-4,5-diamino-pyrazol, 4,5-Diamino-1-(4'-methylbenzyl)-pyrazol sowie Tetraaminopyrimdin in Betracht.

Die Entwicklersubstanzen können sowohl alleine als auch im Gemisch miteinander eingesetzt werden, wobei die Gesamtmenge der Entwicklersubstanzen etwa 0,01 bis 10 Gewichtsprozent, vorzugsweise etwa 0,05 bis 5 Gewichtsprozent, betragen soll.

Als Kupplersubstanzen können in dem erfindungsgemäßen Haarfärbemittel insbesondere Resorcin, 4-Chlorresorcin, 4,6-Dichlorresorcin, 2-Methylresorcin, 2-Amino-4-(2'-hydroxyethylamino)anisol, 2,4-Diamino-5-fluor-toluol, m-Phenylendiamin, 5-Amino-2-methylphenol, 2,4-Diaminophenoxyethanol, 1-Naphthol, m-Aminophenol, 3-Amino-4-chlor-6-methylphenol, 3-Amino-2-methylphenol, 4-Amino-2-hydroxyphenoxyethanol, 4-Amino-5-fluor-2-hydroxytoluol, 4-Amino-5-ethoxy-2-hydroxy-toluol, 4-Hydroxy-1,2-methylendioxybenzol, 4-(2'-Hydroxyethylamino)-1,2-methylendioxybenzol, 2,4-Diamino-5-ethoxytoluol, 4-Hydroxyindol und 3,5-Diamino-2,6-dimethoxypyridin, jeweils alleine oder im Gemisch miteinander verwendet werden.

Die Kupplersubstanzen werden in einer Gesamtmenge von etwa 0,01 bis 5 Gewichtsprozent, vorzugsweise etwa 0,05 bis 3 Gewichtsprozent, in dem erfindungsgemäßen Haarfärbemittel eingesetzt.

Die Gesamtmenge der in dem hier beschriebenen Haarfärbemittel enthaltenen Entwicklersubstanz-Kupplersubstanz-Kombination beträgt vorzugsweise etwa 0,1 bis 6 Gewichtsprozent, wobei eine Menge von etwa 0,5 bis 4 Gewichtsprozent besonders bevorzugt ist. Die Entwicklerkomponente wird im allgemeinen in einer etwa äquimolaren Menge, bezogen auf die Kupplerkomponente, eingesetzt. Es ist jedoch nicht nachteilig, wenn die Entwicklerkomponente diesbezüglich in einem gewissen Überschuß oder Unterschuß vorhanden ist.

Weiterhin kann das erfindungsgemäße Haarfärbemittel zusätzlich andere Farbkomponenten, beispielsweise 6-Amino-2-methylphenol und 2-Amino-5-methylphenol, sowie ferner übliche direktziehende Farbstoffe, zum Beispiel Triphenylmethanfarbstoffe, wie zum Beispiel Basic Violet 14 (C.I. 42 510) und Basic Violet 2 (C.I. 42 520), aromatische Nitrofarbstoffe, beispielsweise 2-Amino-4,6-dinitrophenol, 2-Amino-5-(2'-hydroxyethyl - amino)-nitrobenzol, 4-(2'-Hydroxyethylamino)-3-nitrotoluol und 1-(2'-Ureidoethyl)amino-4-nitrobenzol, Azofarbstoffe, wie zum Beispiel Acid Brown 4 (C.I. 14 805), und Dispersionsfarbstoffe, wie beispielsweise 1,4-Diaminoanthrachinon und 1,4,5,8-Tetraaminoanthrachinon, enthalten. Die Haarfärbemittel können diese Farbkomponenten in einer Menge von etwa 0,1 bis 4,0 Gewichtsprozent enthalten.

Weitere geeignete direkt auf das Haar aufziehende Farbstoffe sind unter anderem in dem Buch von J.C. Johnson "Hair Dyes", Noyes Data Corp., Park Ridge, USA (1973), Seiten 3 bis 91 und 113 bis 139 (ISBN: 0-8155-0477-2) beschrieben, auf welches hiermit ausdrücklich Bezug genommen wird.

Selbstverständlich können die Kuppler- und Entwicklersubstanzen sowie die anderen Farbkomponenten - sofern es Basen sind - auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen

- in Form der Salze mit Basen, zum Beispiel als Alkaliphenolate, eingesetzt werden.

Vorzugsweise enthält das Haarfärbemittel zusätzlich Antioxidantien, wie zum Beispiel Ascorbinsäure, Natriumbisulfit, Natriumsulfit oder Thioglykolsäure, in einer Menge von etwa 0,1 bis 1,5 Gewichtsprozent, wobei die Verwendung von Ascorbinsäure, Natriumsulfit und insbesondere Natriumbisulfit besonders bevorzugt ist.

Die Zubereitungsform des neuen Haarfärbemittels kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion.

Ihre Zusammensetzung stellt eine Mischung der Farbstoffkomponenten mit den für solchen Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol, Isopropanol und Glycerin, oder Glykole, beispielsweise 1,2-Propylenglykol, weiterhin Netzmittel oder Emulgatoren aus den Klassen der anionischen, kationischen, amphoteren oder nichtionogenen oberflächenaktiven Substanzen, beispielsweise Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke, Cellulosederivate, Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Haarfärbemittel schwach sauer, neutral oder alkalisch reagieren. Insbesondere weist es einen pH-Wert von 6,0 bis 11,5 auf, wobei die Einstellung vorzugsweise mit Ammoniak erfolgt. Es können aber auch organische Amine, zum Beispiel Monoethanolamin und Triethanolamin, oder auch organische Basen wie Natriumhydroxid und Kaliumhydroxid Verwendung finden. Für eine pH-Einstellung im sauren Bereich kommen zum Beispiel Phosphorsäure, Essigsäure, Zitronensäure oder Weinsäure in Betracht.

Für die Anwendung zur oxidativen Färbung von Haaren vermischt man das vorstehend beschriebene Haarfärbemittel üblicherweise vor dem Gebrauch mit einem Oxidationsmittel und trägt eine für die Haarfärbebehandlung ausreichende Menge, je nach Haarfülle im allgemeinen etwa 60 bis 200 Gramm, dieses Gemisches auf das Haar auf.

Als Oxidationsmittel zur Entwicklung der Haarfärbung kommen vorzugsweise Wasserstoffperoxid oder dessen Additionsverbindungen an Harnstoff, Melamin oder Natriumborat in Form einer 3- bis 12-prozentigen, vorzugsweise einer 6-prozentigen, wäßrigen Lösung, aber auch Luftsauerstoff in Betracht. Wird eine 6-prozentige Wasserstoffperoxidlösung als Oxidationsmittel verwendet, so beträgt das Gewichtsverhältnis vorzugsweise 5:1 bis 1:2, wobei ein Verhältnis von 1:1 besonders bevorzugt ist. Größere Mengen an Oxidationsmittel werden vor allem bei höheren Farbstoffkonzentrationen im Haarfärbemittel oder wenn gleichzeitig eine stärkere Bleichung der Haare beabsichtigt ist verwendet.

Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Das erfindungsgemäße Haarfärbemittel führt zu Haarfärbungen mit ausgezeichneten Echtheitseigenschaften, insbesondere was die Licht-, Wasch- und Reibeechtheit anbetrifft, und läßt sich mit Reduktionsmitteln wieder abziehen. Hinsichtlich der färberischen Möglichkeiten bietet das erfindungsgemäße Haarfärbemittel je nach Art und Zusammensetzung der Farbkomponenten eine breite Palette verschiedener Farbnuancen, insbesondere auch im Bereich der modischen Goldnuancen. Schließlich ist mit Hilfe des beschriebenen Haarfärbemittels auch eine Anfärbung von ergrautem und chemisch nicht vorgeschädigtem Haar problemlos und mit sehr guter Deckkraft möglich. Die erhaltenen Färbungen sind unabhängig von der unterschiedlichen Struktur des Haares gleichmäßig und trotz der in der Praxis bei den Färbebedingungen auftretenden Abweichungen sehr gut reproduzierbar, ohne daß die üblicherweise beobachteten Farbverschiebungen auftreten.

Die Azofarbstoffe der Formel (I) sind durch einfache chemische Reaktionen, beispielsweise gemäß den in der DE-PS 543 288 oder der DE-OS 4 219 738 beschriebenen Verfahren, in guten Ausbeuten herstellbar, und weisen eine hervorragende Stabilität gegenüber Reduktionsmitteln auf.

Die nachfolgenden Beispiele sollen den Gegenstand der Erfindung näher erläutern ohne diesen hierauf zu beschränken.

## Beispiele

| Beispiel 1: Haarfärbegel | |
|---|---|
| 1,0 g | 4-Aminophenol |
| 0,4 g | α-Naphthol |
| 0,4 g | 3-Amino-6-methyl-phenol |
| 0,3 g | 2,6-Diamino-3-[3'-azopyridyl]-pyridin |
| 0,1 g | Resorcin |
| 0,1 g | 2,5-Diaminotoluol-sulfat |
| 15,0 g | Ölsäure |
| 8,0 g | Isopropanol |
| 6,0 g | Ammoniak (25-prozentige wäßrige Lösung) |
| 3,0 g | Glycerin |
| 0,3 g | Ascorbinsäure |
| 0,1 g | Natriumbisulfit |
| 65,3 g | Wasser |
| 100,0 g | |

20 g des Haarfärbegels werden mit 20 g einer 9-prozentigen Wasserstoffperoxidlösung vermischt. Anschließend wird das so erhaltene gebrauchsfertige Haarfärbemittel auf graues Haar aufgetragen und 30 Minuten lang bei 40 Grad Celsius einwirken gelassen. Sodann wird das Haar mit Wasser ausgespült und getrocknet.
Es wird eine dunkelgoldblonde Färbung erhalten.
Wird in dem vorgenannten Haarfärbemittel das 2,6-Diamino-3-[3'-azopyridyl]-pyridin mengengleich durch Wasser ersetzt, so wird unter den beschriebenen Färbebedingungen eine stumpfe und leblose, wenig ansprechende Färbung erhalten.

| Beispiel 2: Haarfärbecreme | |
|---|---|
| 0,32 g | 2,5-Diaminotoluol-sulfat |
| 0,30 g | 4-Amino-4'-[N,N-bis-(2"-Hydroxyethyl)amino]azobenzol |
| 0,20 g | 2-Amino-6-chlor-4-nitrophenol |
| 0,18 g | 2,6-Dihydroxytoluol |
| 15,00 g | einer Mischung aus Cetylstearylalkohol und Natriumlaurylsulfat (90:10) (Lanette® W der Firma Henkel KGaA/BRD) |
| 3,50 g | Natriumdiglykollaurylethersulfat (20-prozentige wäßrige Lösung) |
| 2,40 g | Natronlauge (20-prozentige wäßrige Lösung) |
| 0,40 g | Natriumsulfit |
| 0,20 g | Ascorbinsäure |
| 0,20 g | Monoethanolamin |
| 77,30 g | Wasser |
| 100,00 g | |

10 g der obigen Haarfärbecreme werden mit 20 g einer 2-prozentigen Wasserstoffperoxidlösung zu einer gebrauchsfertigen Haarfärbezubereitung mit einem pH-Wert von 6,8 vermischt. Anschließend wird graues Haar in der in Beispiel 1 beschriebenen Weise gefärbt. Als Ergebnis der Färbebehandlung wird eine glänzende, mittelblonde, goldschimmernde Nuance erhalten.
Alle Prozentangaben stellen, sofern nicht anders angegeben ist, Gewichtsprozente dar.

## Patentansprüche

**1.** Mittel zur oxidativen Färbung von Haaren, auf der Basis einer Entwicklersubstanz/Kupplersubstanz-Kombination, dadurch gekennzeichnet, daß es mindestens einen Azofarbstoff der Formel (I)

$$A - N = N - B \qquad (I),$$

in der A einen p-Aminophenylrest der Formel (II) oder einen 3-Pyridylrest der Formel (III) darstellt

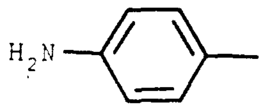 (II) 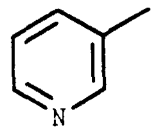 (III)

und B einen 4-[N,N-Bis-(2'-hydroxyethyl)amino]phenylrest der Formel (IV) oder einen 2,6-Diamino-3-pyridylrest der Formel (V)

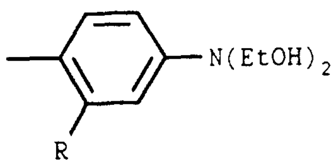 (IV),

wobei R gleich Wasserstoff, einer Alkylgruppe mit 1 bis 6 Kohlenstoffatomen, Fluor, Chlor, Brom oder Jod ist,

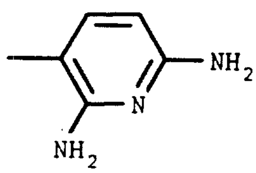 (V)

bedeutet, enthält.

**2.** Mittel nach Anspruch 1, dadurch gekennzeichnet, daß der Azofarbstoff ausgewählt ist aus Verbindungen der Formel (I) bei denen der Rest A eine Gruppe gemäß Formel (II) oder (III) darstellt, wenn der Rest B gleich Formel (V) ist und der Rest A eine Gruppe gemäß Formel (II) darstellt wenn der Rest B gleich Formel (IV) ist.

**3.** Mittel nach Anspruch 1 oder 2, dadurch gekennzeichnet, daß der Azofarbstoff der Formel (I) ausgewählt ist aus 4-Amino-4' -[N,N-bis- (2''-hydroxyethyl) amino]azobenzol, 4-Amino-2'-methyl-4'-[N,N-bis-(2''-hydroxyethyl) amino] azobenzol, 4-Amino-2'-chlor-4'-[N,N-bis-(2''-hydroxyethyl)amino]-azobenzol und 2,6-Diamino-3-[3'-azopyridyl]-pyridin.

**4.** Mittel nach einem der Ansprüche 1 bis 3, dadurch gekennzeichnet, daß der Azofarbstoff der Formel (I) in einer Menge von 0,01 bis 2 Gewichtsprozent enthalten ist.

**5.** Mittel nach einem der Ansprüche 1 bis 4, dadurch gekennzeichnet, daß die Entwicklersubstanz in einer Menge von 0,01 bis 10 Gewichtsprozent enthalten ist.

**6.** Mittel nach einem der Ansprüche 1 bis 5, dadurch gekennzeichnet, daß die Kupplersubstanz in einer Menge von 0,01 bis 5 Gewichtsprozent enthalten ist.

**7.** Mittel nach einem der Ansprüche 1 bis 6, dadurch gekennzeichnet, daß die Entwicklersubstanz/Kupplersubstanz-Kombination in einer Menge von 0,1 bis 6 Gewichtsprozent enthalten ist.

**8.** Mittel nach einem der Ansprüche 1 bis 7, dadurch gekennzeichnet, daß es ein Reduktionsmittel enthält.

**9.** Mittel nach Anspruch 8, dadurch gekennzeichnet, daß das Reduktionsmittel ausgewählt ist aus Ascorbinsäure, Natriumsulfit und Natriumbisulfit.

**10.** Mittel nach Anspruch 8 oder 9, dadurch gekennzeichnet, daß das Reduktionsmittel in einer Menge von 0,1 bis 1,5 Gewichtsprozent enthalten ist.

**11.** Verfahren zur oxidativen Färbung von Haaren, dadurch gekennzeichnet, daß man vor dem Gebrauch ein Haarfärbemittel nach einem der Ansprüche 1 bis 10 mit einem Oxidationsmittel vermischt, sodann eine für die Haarfärbung ausreichende Menge dieses Gemisches auf das Haar aufträgt, es dort 10 bis 45 Minuten bei 15 bis 50 Grad Celsius einwirken läßt, anschließend das Haar mit Wasser spült und sodann trocknet.

**12.** Verfahren nach Anspruch 11, dadurch gekennzeichnet, daß das Oxidationsmittel ausgewählt ist aus Wasserstoffperoxid und dessen Additionsverbindungen an Harnstoff, Melamin oder Natriumborat.

**13.** Verfahren nach Anspruch 11 oder 12, dadurch gekennzeichnet, daß als Oxidationsmittel eine 6-prozentige Wasserstoffperoxidlösung verwendet wird und das Haarfärbemittel mit dem Oxidationsmittel in einem Gewichtsverhältnis von 5:1 bis 1:2 vermischt wird.

**14.** Verfahren nach Anspruch 11, dadurch gekennzeichnet, daß als Oxidationsmittel Luftsauerstoff verwendet wird.

**Claims**

**1.** Agent for the oxidative dyeing of hair, based on a developer substance/coupler substance combination, characterised in that it contains at least one azo dye of formula (I)

$$A - N = N - B \qquad (I)$$

in which A represents a p-aminophenyl residue of formula (II) or a 3-pyridyl residue of formula (III)

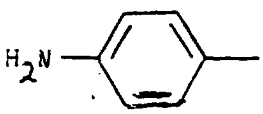 (II) 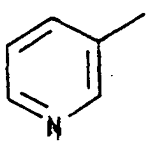 (III)

and B represents a 4-[N,N-bis-(2'-hydroxyethyl)amino]-phenyl residue of formula (IV) or a 2,6-diamino-3-pyridyl residue of formula (V)

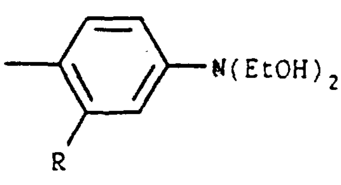 (IV),

wherein R is hydrogen, an alkyl group having from 1 to 6 carbon atoms, fluorine, chlorine, bromine or iodine,

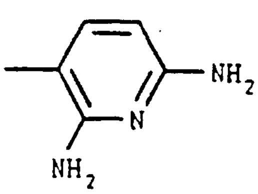 (V)

**2.** Agent according to claim 1, characterised in that the azo dye is selected from compounds of formula (I) wherein the residue A is a group according to formula (II) or (III) when the residue B represents the formula (V) and the residue A represents a group according to formula (II) when the residue B represents the formula (IV).

**3.** Agent according to claim 1 or 2, characterised in that the azo dye of formula (I) is selected from 4-amino-4'-[N,N-bis-(2"-hydroxyethyl)amino]-azobenzene, 4-amino-2'-methyl-4'-[N,N-bis-(2"-hydroxyethyl)amino]azobenzene, 4-amino-2'-chloro-4'-[N,N-bis-(2"-hydroxyethyl)amino]-azobenzene and 2,6-diamino-3-[3'-azopyridyl]-pyridine.

**4.** Agent according to any one of claims 1 to 3, characterised in that the azo dye of formula (I) is present in an amount of from 0.01 to 2% by weight.

**5.** Agent according to any one of claims 1 to 4, characterised in that the developer substance is present in an amount of from 0.01 to 10% by weight.

**6.** Agent according to any one of claims 1 to 5, characterised in that the coupler substance is present in an amount of from 0.01 to 5% by weight.

**7.** Agent according to any one of claims 1 to 6, characterised in that the developer substance/coupler substance combination is present in an amount of from 0.1 to 6% by weight.

**8.** Agent according to any one of claims 1 to 7, characterised in that it contains a reducing agent.

**9.** Agent according to claim 8, characterised in that the reducing agent is selected from ascorbic acid, sodium sulphite and sodium bisulphite.

**10.** Agent according to claim 8 or 9, characterised in that the reducing agent is present in an amount of from 0.1 to 1.5% by weight.

**11.** Process for the oxidative dyeing of hair, characterised in that, prior to use, a hair dyeing agent according to any one of claims 1 to 10 is mixed with an oxidising agent, then an amount of that mixture sufficient to dye the hair is applied to the hair, is left there to take effect for from 10 to 45 minutes at from 15 to 50°C, and the hair is subsequently rinsed with water and then dried.

**12.** Process according to claim 11, characterised in that the oxidising agent is selected from hydrogen peroxide and its addition compounds to urea, melamine or sodium borate.

**13.** Process according to claim 11 or 12, characterised in that a 6% hydrogen peroxide solution is used as the oxidising agent and the hair dyeing agent is mixed with the oxidising agent in a ratio by weight of from 5:1 to 1:2.

**14.** Process according to claim 11, characterised in that atmospheric oxygen is used as the oxidising agent.

**Revendications**

**1.** Agent de teinture oxydative des cheveux, à base d'une combinaison développeur-copulateur, caractérisé en ce qu'il contient au moins un colorant azo de formule (I)

$$A - N = N - B \qquad (I)$$

dans lequel A est un résidu p-aminophényle de formule (II) ou un résidu 3-pyridyle de formule (III)

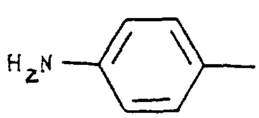 (II) 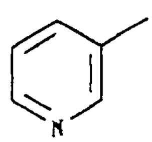 (III)

et B est un résidu 4-[N,N-bis-(2'-hydroxyéthyl)amino]phényle de formule (IV) ou un résidu 2,6-diamino-3-pyridyle de formule (V)

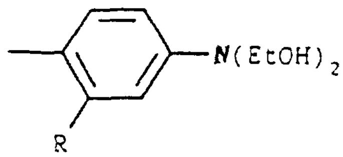

(IV),

R étant l'hydrogène, un groupe alkyle ayant de 1 à 6 atomes de carbone, le fluor, le chlore, le brome ou l'iode

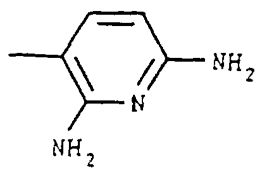

(V)

2. Agent selon la revendication 1, caractérisé en ce que le colorant azo est sélectionné parmi des composés de la formule (I) pour lesquelles le résidu A constitue un groupe répondant à la formule (II) ou à la formule (III) lorsque le résidu B est de la formule (V), et le résidu A est un groupe répondant à la formule (II) lorsque le résidu B est de la formule (IV).

3. Agent selon la revendication 1 ou 2, caractérisé en ce que le colorant azo de formule (I) est sélectionné parmi le 4-amino-4'-[N,N-bis-(2''-hydroxyéthyl)amino]-azobenzène, le 4-amino-2'méthyl-4'-[N,N-bis-(2''-hydroxyéthyl)-amino]-azobenzène, le 4-amino-2'-chloro-4'-[N,N-bis-(2''-hydroxyéthyl)-amino]-azoben-zène et le 2,6-diamino-3-[3'-azopyridyl]-pyridine.

4. Agent selon l'une des revendications 1 à 3, caractérisé en ce que le colorant azo de formule (I) est contenu en une quantité comprise dans la plage allant de 0,01 à 2 pour-cent en poids.

5. Agent selon l'une des revendications 1 à 4, caractérisé en ce que le développeur est contenu en une quantité comprise dans la plage allant de 0,01 à 10 pour-cent en poids.

6. Agent selon l'une des revendications 1 à 5, caractérisé en ce que le copulateur est contenu en une quantité comprise dans la plage allant de 0,01 à 5 pour-cent en poids.

7. Agent selon l'une des revendications 1 à 6, caractérisé en ce que la combinaison développeur/copulateur est contenue en une quantité comprise dans la plage allant de 0,1 à 6 pour-cent en poids.

8. Agent selon l'une des revendications 1 à 7, caractérisé en ce qu'il contient un agent de réduction.

9. Agent selon la revendication 8, caractérisé en ce que l'agent de réduction est sélectionné parmi l'acide ascorbique, le sulfite de sodium et le bisulfite de sodium.

10. Agent selon la revendication 8 ou 9, caractérisé en ce que l'agent de réduction est contenu en une quantité comprise dans la plage allant de 0,1 à 1,5 pour-cent en poids.

11. Procédé de teinture oxydative des cheveux, caractérisé en ce que, avant utilisation, on mélange un agent de teinture capillaire selon l'une des revendications 1 à 10 avec un agent d'oxydation, puis on applique une quantité suffisante pour la teinture capillaire de ce mélange sur les cheveux, on l'y laisse agir de 10 à 45 minutes à une température comprise dans la plage allant de 15 à 50 degrés Celsius, puis on rince les cheveux à l'eau et l'on sèche ensuite.

12. Procédé selon la revendication 11, caractérisé en ce que l'on choisit l'agent d'oxydation parmi le peroxyde d'hydrogène et ses combinaisons d'addition sur l'urée, la mélamine ou le borate de sodium.

**13.** Procédé selon la revendication 11 ou 12, caractérisé en ce que l'on utilise comme agent d'oxydation une solution de peroxyde d'hydrogène a 6 pour-cent et l'agent de teinture capillaire est mélangé à l'agent d'oxydation en un rapport de poids allant de 5:1 à 1:2.

**14.** Procédé selon la revendication 11, caractérisé en ce que l'on utilise comme agent d'oxydation l'oxygène de l'air.